# EUROPEAN PATENT APPLICATION

(11) **EP 2 279 993 A1**
(43) Date of publication of application: **02.02.2011**
(21) Application number: 10177167.3
(22) Date of filing: 20.11.2002
(51) Int. Cl.: C07C 39/15, C07C 37/11

(54) **Process for preparing 3,3',5,5',6,6'-hexaalkyl-2,2'-biphenols, 3,3',4,4',5,5'-hexaalkyl-2,2'-biphenols and 3,3',4,4',5,5',6,6'-octaalkyl-2,2'-biphenols**

(30) Priority: 26.11.2001 US 994133
(62) Divisional of application: 02804010.3
(71) Applicant: INVISTA Technologies S.à.r.l., 8001 Zürich (CH)
(72) Inventor: Shapiro, Rafael, Wilmington, DE 19803 (US)
(74) Representative: Cockerton, Bruce Roger

(57) **Abstract**

A process for making a compound of Formula (I) by oxidative coupling of a compound of the formula (11) in the presence of a molecular oxygen-containing gas and a copper-containing catalyst produced by contacting a copper halide salt with an organic diamine compound. Some compounds of the formula (I) are also claimed.

## Description

### FIELD OF THE INVENTION

This invention relates to a process for preparing 3,3',4,4',5,5',6,6'-octaalkyl-2,2'-biphenols, 3,3',4,4',5,5'-hexaalkyl-2,2'-biphenols and 3,3',5,5',6,6'-hexaalkyl-2,2'-biphenols.

### BACKGROUND OF THE INVENTION

Substituted biphenols such as 3,3',6,6'-tetraalkyl-2,2'-biphenol; 3,3',4,4',5,5'-hexaalkyl-2,2'-biphenols; 3,3',4,4', 5,5',6,6'-octaalkyl-2,2'-biphenols; 3,3',5,5',6,6'-hexaalkyl-2,2'-biphenols; 3,3',5,5'-tetraalkyl-2,2'-biphenol; 3-alkyl-5,5',6,6',7,7'8,8'-octahydro-2,2'-binaphthol; 3,3'-dialkyl-5,5',6,6',7,7'8,8'-octahydro-2,2'-binaphthol and 3,3'6,6'-tetralkyl-5,5'-dihalo-2,2'-biphenol are compounds that can be used to make phosphorus-based catalyst ligands. Such ligands include phosphines, phosphinites, phosphonites, and phosphites. Mono(phosphorous) ligands are compounds that contain a single phosphorus atom which serves as a donor to a transition metal, while bis(phosphorus) ligands, in general, contain two phosphorus donor atoms and typically form cyclic chelate structures with transition metals.

In general, biphenols can be made by the oxidative coupling of (mono)phenols, but often other types of products, such as ketones, are obtained, and/or overall yields are poor for other reasons.

Phenols can be oxidatively coupled to make the corresponding biphenols by the use of a variety of oxidizing agents, such as nitric acid, ferric chloride, potassium ferricyanide, chromic acid, 2,3-dichloro-5,6-dicyanobenzoquinone and di-t-butyl peroxide. 2,2'-Dihydroxy-3,3'-di-isopropyl-6,6'-dimethylbiphenyl can be prepared from 2-isopropyl-5-methyl-phenol with 2,3-dichloro-5,6-dicyanobenzoquinone or di-t-butyl peroxide. See Tetrahedron, 1875, 1971 and J. Chem. Soc., Perkin Trans. II, 587, 1983. Some of the oxidants and/or cocatalysts involve the use of relatively expensive and/or explosive (peroxides) compounds, which pose disadvantages for large scale commercial use.

Phenols can also be oxidatively coupled using a combination of a transition metal catalyst and an oxidizing agent such as persulfate anion or oxygen. See U.S. Patents 6,077,979, 4,139,544, 4,132,722, 4,354,048, and 4,108,908, J. Org. Chem. 1984, 49, 4456 and J. Org. Chem. 1983, 48, 4948. The cited patents disclose the use of oxygen as an oxidizing agent with various catalytic copper complexes such as copper chromite, copper acetate with sodium mercaptoacetate, copper acetate with pentasodium/diethylenetriaminepentacetate; and copper acetate with 1,3-diamino-2-hydroxypropane-tetracetic acid. The examples in the patents disclose the use of 2,6-disubstituted phenol or 2,4-di-tert-butylphenol.

The use of copper amine catalysts, with oxygen as an oxidizing agent, has been described in connection with the oxidative coupling of 2,4-di-tert-butylphenol, 2-methyl-4-tert-butylphenol, 2-chlor-4-tert-butylphenol and 4-tert-butylphenol See, J. Org. Chem. 1984, 49, 4456 and J. Org. Chem. 1983, 48, 4948.

There is a continuing need in the art for methods for making with decent yields substituted biphenols suitable for making phosphorous-based catalyst ligands.

### SUMMARY OF THE INVENTION

In a first aspect, the present invention is a process for making a compound of the formula 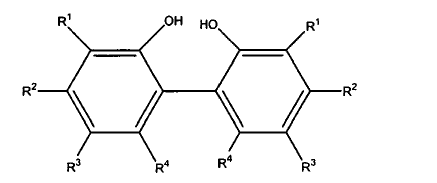 comprising:
oxidatively coupling a compound of the formula 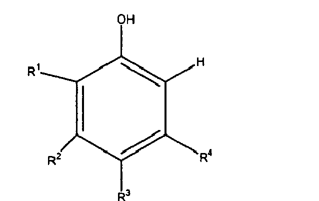 in the presence of a molecular oxygen-containing gas and a copper-containing catalyst, said copper-containing catalyst produced by a process comprising contacting a copper halide salt with an organic diamine compound, wherein
   R1 is C1 to C6 primary, secondary or cyclo alkyl;
   R2 is H, C1 to C6 primary, secondary, tertiary or cyclo alkyl;
   R3 is C1 to C6 primary, secondary, tertiary or cyclo alkyl;
   R4 is H, C1 to C6 primary, secondary or cyclo alkyl, provided that R2 and R4 are not both H.

In a second aspect, the present invention is a compound of the formula 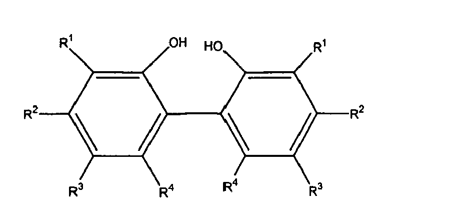 wherein:
R¹ is methyl, ethyl, n-propyl, or isopropyl;
R² is H or methyl;
R³ is methyl, ethyl, n-propyl, isopropyl, or t-butyl; and
R⁴ is methyl;
   provided that if R¹ is isopropyl and R² is hydrogen, R³ is other than methyl.

Preferred compounds are those described above wherein
R¹ is methyl or isopropyl;
R² is H or methyl;
R³ is methyl, isopropyl, or t-butyl; and
R⁴ is methyl.

Most preferred are compounds of the immediately preceeding paragraph wherein
R¹ is isopropyl;
R² is H;
R³ is isopropyl; and
R⁴ is methyl.

### DETAILED DESCRIPTION OF THE INVENTION

The present invention provides a process for preparing 3,3',5,5',6,6'-hexaalkyl-2,2'-biphenol, 3,3',4,4',5,5'-hexaalkyl-2,2'-biphenol, or 3,3',4,4',5,5',6,6'-ocatalkyl-2,2'-biphenol by oxidatively coupling 2,4,5-trialkylphenol, 2,3,4-trialkylphenol, or 2,3,4,5-tetraalkylphenol, respectively, with a copper amine catalyst and oxygen as oxidizing agent. Suitable phenols are represented by the formula 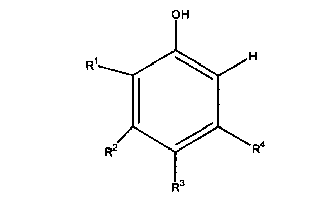 wherein R¹ is C₁ to C₆ primary, secondary or cyclo alkyl;
R² is H, C₁ to C₆ primary, secondary, tertiary or cyclo alkyl;
R³ is C₁ to C₆ primary, secondary, tertiary or cyclo alkyl;
R⁴ is H, C₁ to C₆ primary, secondary or cyclo alkyl; provided that R² and R⁴ are not both H.

The alkyl groups can be linked together or unlinked. For example, alkyl groups, R¹ and R² , can be connected to form fused cyclic alkyl groups. Similarly, alkyl groups R² and R³, or R³ and R⁴ can be connected to form fused cyclic alkyl groups. Some representative 2,4,5-trialkylphenols, 2,3,4-trialkylphenols and 2,3,4,5-tetraalkylphenols, are those shown in the following formulas.

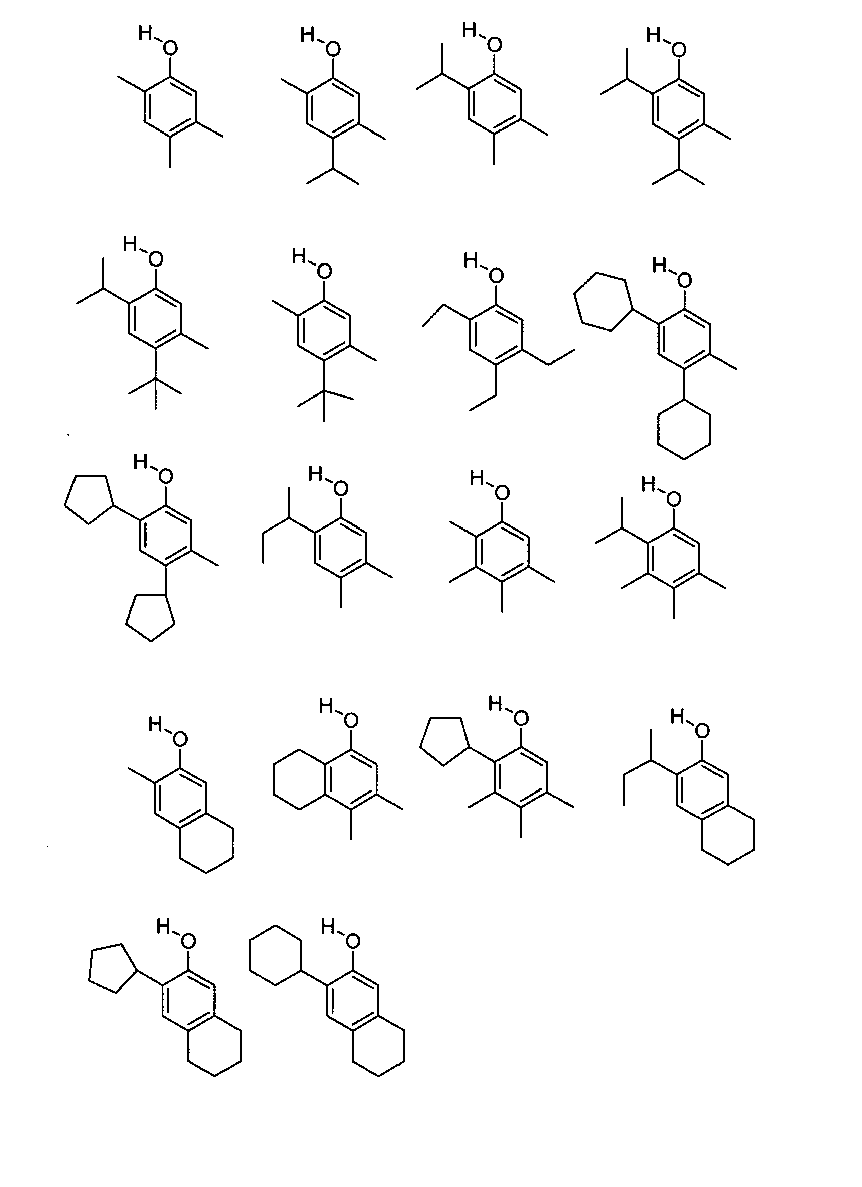

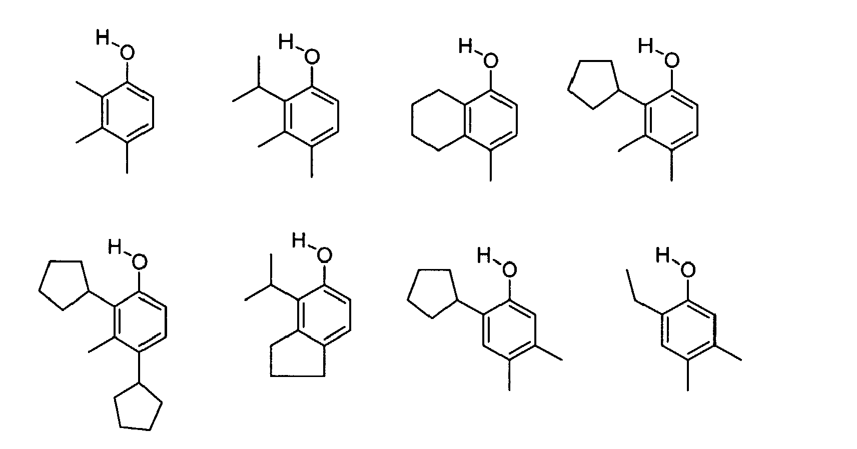

Dimerization of 2,4,5-trialkylphenols, 2,3,4-trialkylphenols, 2,3,4,5-tetraalkylphenols or 2,4-dialkylphenols by oxidative coupling leads to the corresponding biphenols. The oxidative coupling can be carried out neat (without a solvent) or with one or more of a wide range of poorly oxidizable solvents including dichloromethane, chlorobenzene, toluene, xylenes, nitromethane, paraffins, etc. A molecular oxygen-containing gas is used as the oxidant. For example, static air, flowing air, or oxygen can be used in the oxidative coupling. The reaction is typically carried out by contacting the phenol with a copper complex of a diamine in an inert, preferably aprotic solvent such as dichloromethane, toluene, chlorobenzene, or saturated hydrocarbon, preferably one having a flash-point higher than the reaction temperature, at a temperature between 5 and 100°C, preferably around 30°C. The product is generally isolated by dilution with a saturated hydrocarbon solvent, filtration, and optionally purified by washing with aqueous mineral acid or a copper-sequestering reagent such as sodium EDTA. The biphenol may optionally be purified by recrystallization.

The copper diamine catalyst can be prepared using the procedure described in Tetrahedron Letters, 1994, 35, 7983. A copper halide, such as CuCl, CuBr, CuI, CuCl2, is added to a mixture of alcohol, such as methanol, and water and the diamine is slowly added. After the addition of the diamine, air is sparged through the mixture with vigorous stirring. The catalyst is filtered. Additional catalyst can be obtained by concentrating the filtrate and filtering the desired catalyst. The catalyst can also be prepared in situ by contacting the copper halide and the diamine in the solvent for the coupling reaction. Example of diamines include, but are not limit to, the following: N,N,N',N'-tetraethylethylene diamine, N,N,N',N'-tetraethyl-1,3-propanediamine, N,N,N',N'-tetraethylmethane diamine, N,N,N',N'-tetramethyl-1,6-hexanediamine, N,N,N',N'-tetramethyl-1,3-propanediamine, dipiperidinomethane, N,N,N',N'-tetramethylethylene diamine and 1,4-diazabicyclo-(2,2,2)-octane. Preferrably, the diamines are N,N,N',N'-tetrasubstituted ethylenediamine or propylenediamine or methylenediamine, such as tetramethylethylenediamine (TMEDA), N,N,N',N'-tetraethyl-1,3-propanediamine and N,N,N',N'-tetraethylmethane diamine. The 3,3',5,5',6,6'-hexaaklylphenols made by the processes of the present invention can be used to make polymeric ligands by a process which comprises: (1) reacting the 3,3',5,5',6,6'-hexaalkylphenols made by the processes of the present invention with a benzyl chloride group-containing polymer in the presence of a Lewis acid catalyst, and (2) reacting the product of step (1) with at least one phosphorochloridite compound in the presence of an organic base. Preferably the Lewis acid catalyst is aluminum chloride, and the organic base is a trialkylamine.

The biphenols of the present invention can used to produce bidentate phosphite compounds. Preparation of bidentate phosphites using biphenols are described in U.S. Patents 5,235,113, 6,031,120 and 6,069,267, the disclosures of which are incorporated herein by reference. Two industrially important processes that utilize bidentate phosphite compounds are the hydrocyanation and hydroformylation of olefinic compounds. Bidentate phosphite compounds have been shown to be useful in the hydrocyanation of monoolefinic and diolefinic compounds, as well as for the isomerization of non-conjugated 2-alkyl-3-monoalkenenitriles to 3- and/or 4-monoalkene.
See, for example, U.S. Patents 5,512,695, 5,512,696, and International Patent Application WO9514659. Bidentate phosphite ligands have also been shown to be useful in olefin hydroformylation reactions. See for example, U.S. Patent 5,235,113.

The present invention also relates to compounds of the formula 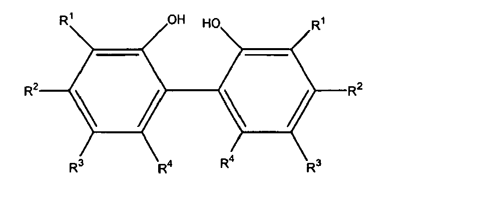 wherein:
R¹ is methyl, ethyl, n-propyl, or isopropyl;
R² is H or methyl;
R³ is methyl, ethyl, n-propyl, isopropyl, or t-butyl; and
R⁴ is methyl;
provided that if R¹ is isopropyl and R² is hydrogen, R³ is
other than methyl.

Preferred compounds are those described above wherein
R¹ is methyl or isopropyl;
R² is H or methyl;
R³ is methyl, isopropyl, or t-butyl; and
R⁴ is methyl.

Most preferred are compounds of the immediately
preceeding paragraph wherein
R¹ is isopropyl;
R² is H;
R³ is isopropyl; and
R⁴ is methyl.

### EXAMPLES

The following non-limiting examples illustrate the present invention.

### Example 1

### Preparation of 5,5'-Bis(t-butyl)-3,3',6,6'-tetramethyl-2,2'-biphenol

To a solution of 18.6 g (0.104 mol) of 4-t-butyl-2,5-xylenol in 20 mL of dichloromethane was added 0.6 g (3 mmol) of copper chlorohydroxide-TMEDA complex (TMEDA = tetramethylethylenediamine). The dark purple mixture was stirred under_ambient air overnight. Gas chromatography (GC) analysis showed only 25% conversion, so the mixture was diluted with dichloromethane, dried (MgSO₄) and concentrated to dryness. To the crude residue was added 20 mL of cyclohexane and 1.2 g (6 mmol) of the above copper chlorohydroxide-TMEDA catalyst, and the mixture was stirred under air at ambient temperature for three days (85% conversion). The purple solution was concentrated to dryness, and the residue was chromatographed on silica gel to give 10.2 g (55%) of pure 5,5'-Bis(t-butyl)-3,3',6,6'-tetramethyl-2,2'-biphenol , mp 103-105°C. ¹H-NMR (CDCl₃) 1.42, (s, 9H), 2.06 (s, 3H), 2.25 (s, 3H), 4.54 (s, 1H), 6.51 (s, 1H), 7.24 (s, 1H).

### Example 2

### Preparation of 5,5'-Di-t-butyl-3,3'-di-isopropyl,6,6'-dimethyl-2,2'-biphenol

To a solution of 20 g (0.104 mol) of 4-t-butylthymol in 50 mL of dichloromethane was added 1.0 g (5 mmol) of copper chlorohydroxide-TMEDA complex, and the dark purple mixture was allowed to stir under ambient air for three days (50% conversion). The mixture was diluted with hexanes, washed with aqueous EDTA solution, dried (MgSO₄) and concentrated to dryness. The residue was chromatographed on silica gel to give 3.6 g (34% based on conversion) of pure dimer 5,5'-Di-t-butyl-3,3'-diisopropyl,6,6'-dimethyl-2,2'-biphenol, mp 105-108°C. ¹H-NMR (CDCl₃) δ 1.26 (d,6H), (s, 9H), 3.25 (septet, 1H), 4.58 (s, 1H), 7.30 (s, 1H).

### Example 3

### Preparation of 3,3',4,4',5,5',6,6'-octamethyl-2,2'-biphenol

### Preparation of 2,3,4,5-tetramethylphenol

To 56 g of 85%-pure 5-bromoprehnitene (0.22 mol) (prepared according to J. Am. Chem. Soc. 1929, 3001; used acetic acid instead of chloroform as solvent, with 1 wt% of iron powder at ambient temperature and fractionally-distilled the product) in 50 mL of diglyme was added 1.0 g of 2-aminopyridine, 1.1 g of cuprous chloride, and 80 g of 25% NaOMe in methanol, and the mixture was heated with removal of methanol under nitrogen. After 16 hr heating at 120°C, the conversion was 60%, and an additional 0.7 g aminopyridine, 1.0 g CuCl, and 20 g NaOMe solution were added. After 4 hr at 100°C, the conversion was 90%. The mixture was cooled, diluted with 200 mL of hexanes and 100 mL of aq 3% ammonia, and the organic phase was washed with water, dried (MgSO₄), and concentrated to dryness. The crude 5-methoxyprehnitene thus obtained (43.1 g) was heated for 2 days at 100°C with 130 mL of 48% aqueous HBr, diluted with water and hexanes, cooled to 5°C, and the solids were filtered and washed with cold water and hexane. Drying in vacuo provided 22 g of 2,3,4,5-tetramethylphenol. Another 4.5 g was recovered from the filtrate, totaling 26.5 g (80% based on bromide). ¹H-NMR (CDCl₃) δ 2.12 (s, 3H), 2.16 (s, 3H), 2.19 (s, 3H), 2.21 (s, 3H), 4.44 (s, 1H), 6.48 (s, 1H).

### Dimerization of 2,3,4,5-tetramethylphenol

The monomer (2.6 g, 17.3 mmol) was stirred under air with 10 mL of toluene and 0.15 g (6.3 mmol) Cu(OH)Cl-TMEDA for 6 hr at ambient temperature (85% conversion). The mixture was diluted with 5 mL 1N HCl and 20 mL hexanes, stirred for 15 min, and filtered. The solids were combined with a small second crop from the filtrate and suction-dried to afford 1.4 g (54%) of octamethyl-2,2'-biphenol, mp 202°C. ¹H-NMR (CDCl₃) δ 1.90 (s, 3H), 2.20 (s, 3H), 2.22 (s, 3H), 2.26 (s, 3H), 4.60 (s, 1H).

### Example 4:

### Preparation of 3,3'-diisopropyl-5,5',6,6'-tetramethyl-2,2'-biphenol

To a solution of 15.0 g (0.0915 mol) of 4-methyl thymol in 15 mL of dichloromethane was added 0.75 g (3.2 mmol) of copper chlorohydroxide-TMEDA complex. The solution was stirred exposed to the air for 4 to 6 hr at ambient temperature. The mixture was stirred with 5 mL of saturated aqueous disodium EDTA for 10 minutes to decompose Cu-complexes, diluted with 80 mL of hexanes, and the hexane layer was concentrated to dryness. The crude product was recrystallized from hexanes to afford two crops totaling 8.5 g of product (63% yield based on 90% conversion), ¹H-NMR (CDCl₃) δ 1.24 (d, 6H, J = 7 Hz), 1.87 (s, 3H), 2.26 (s, 3H), 3.26 (septet, 1H, J = 7 Hz), 4.6 (s, 1H), 7.06 (s, 1H). The first crop had mp 107°C (lit. US 4880775: mp 106-107.5°C).

### Larger scale preparation:

To a solution of 2-isopropyl-4,5-dimethylphenol (140 g, 0.85 mol) in 140 ml of dichloromethane was added copper chlorohydroxide-TMEDA complex (5 g). The solution was stirred for 20 hr at ambient temperature while air was bubbled through. The mixture was treated with disodium EDTA at room temperature for 30 min. diluted with hexanes (50 mL) and washed with HCl (0.5 N) and water. The solution was then concentrated to give a residue which was further purified by chromatography to afford 2-isopropyl-4,5-dimethylphenol dimer (80 g, 57 %). Another 5g of impure product was also obtained. ¹H NMR 1.28 (d, J = 7 Hz, 12H), 1.90 (s, 6H), 2.30 (s, 6H), 3.29 (septet, J = 7 Hz, 2H), 4.63 (s, 2H), 7.08 (s, 2H) ppm. ¹³C NMR 16,0, 19.90, 22.5, 22.7, 27.1, 122.2, 128.16, 128.6, 132.0, 133.6, 148.9 ppm.

### Example 5

### Preparation of 3,3'-diisopropyl-5,5'-diethyl-6,6'-dimethyl-2,2'-biphenol

To a solution of 23.5 g of 4-ethyl thymol in 50 mL of toluene was added 1.2 g of Cu(OH)Cl-TMEDA, and the mixture was stirred under ambient air for 18 hr (90% conversion, 80% after 6 hr). The product was worked up as above and chromatographed (SiO₂/hexanes) to afford 10.0 g (42%) of dimer, ca 95%-pure by gc analysis, mp 61-64°C. ¹H-NMR (CDCl₃) δ 1.2 (m, 9H), 1.88 (s, 3H), 2.62 (q, 2H, J = 7.5 Hz), 3.27 (septet, 1H), 4.61 (s, 1H), 7.07 (s, 1H).

### Example 6

### Preparation of 3,3',5,5',6,6'-Hexamethyl-2,2'-biphenol

To a solution of 2,4,5-trimethylphenol (1.9 g) in 4 mL of dichloromethane was added copper chlorohydroxide-TMEDA complex (0.2 g). The solution was stirred for 45 h at ambient temperature while air was bubbled through. The mixture was diluted with ether and washed with HCl (2N) and water, respectively. The ether solution was analyzed by GC, which indicated 95% conversion and 72 % selectivity.

### Copper catalyzed coupling of 2,4,5-trimethylphenol

### a) Catalyst solution

Under exclusion of oxygen a solution of 0.550 g of 2,4,5-trimethylphenol in 10 mL CH₂Cl₂ was mixed with 0.924 of (TMEDA)CuCl(OH) to form a deep blue solution.

### b) Coupling:

A solution of 26.6 g 2,4,5-trimethylphenol in 125mL CH₂CL₂ was charged with 2 mL of the copper catalyst solution as described under (a). The solution was stirred at ambient temperature with a slow flow of air over the solution. Another 2 mL and 3 mL of catalyst solution was added after 19 hr and 34 hr, respectively. The molar ratio between catalyst and 2,4,5-trimethylphenol was 1.4%. After 2d GC analysis showed 99% conversion at 98% selectivity. After cooling the reaction mixture to 0°C the product was filtered off and washed with little CH₂Cl₂ to yield 16.5g 3,3',5,5'6,6'-Hexamethyl-2,2'-biphenol. Another 6.2 g of 3,3',5,5'6,6'-Hexamethyl-2,2'-biphenol were isolated from the mother liquor. The purity of the isolated product by GC and NMR was 99%. ¹H nmr (CDCl₃): δ 6.93 (s, 2H), 4.49 (s, 2H), 2.17 (s, 12H), 1.76 (s, 6H).

### Example 7

### Preparation of 3,3'-dicyclohexyl-5,5',6,6'-tetramethyl-2,2'-biphenol

To a solution of 2-cyclohexyl-4,5-dimethylphenol (4.5 g, 22 mmol) in 25 mL of dichloromethane was added copper chlorohydroxide-TMEDA complex (45 mg). The solution was stirred for 3 hr at ambient temperature while air was bubbled through. The mixture was diluted with ether and washed with HCl (2N) and water, respectively. The ether solution was then concentrated to give a residue which was further purified by chromatography to afford starting 2-cyclohexyl-4,5-dimethylphenol (1.35 g) and 3,3'-dicyclohexyl-5,5',6,6'-tetramethyl-2,2'-biphenol (1.8 g, 57% based on consumed 2-cyclohexyl-4,5-dimethylphenol). ¹H NMR 1.27 (m, 2H), 1.39 (m, 8H), 1.75 (m, 2H), 1.84 (s, 6H), 1.86 (m, 8H), 2.22 (s, 6H), 2.85 (m, 2H), 4.52 (s, 2H), 7.04 (s, 2H) ppm. ¹³C NMR 16.1, 19.9, 26.5, 27.1, 33.2, 37.3, 120.2, 128.6, 129.6, 131.3, 133.6, 148.8 ppm.

### Example 8

### Preparation of 3,3'-dicyclopentyl-5,5',6,6'-tetramethyl-2,2'-biphenol

To a solution of 2-cyclopentyl-4,5-dimethylphenol (3.9 g, 21 mmol) in 10 mL of dichloromethane was added copper chlorohydroxide-TMEDA complex (40 mg). The solution was stirred for 3 hr at ambient temperature while air was bubbled through. The catalyst (40 mg each) was added at 1 and 2 hours after reaction started. The mixture was diluted with dichloromethane (50 mL) and washed with HCl (0.5 N) and water. The solution was then concentrated to give a residue which was further purified by chromatography to afford 3,3'-dicyclopentyl-5,5',6,6'-tetramethyl-2,2'-biphenol (2.5 g, 64%). ¹H NMR 1.71 (m, 8H), 1.83 (m, 4H), 1.89 (s, 6H), 2.05 (m, 4H), 2.29 (s, 6H), 3.30 (quintet, J = 7 Hz, 2H), 4.61 (s, 2H), 7.12 (s, 2H) ppm. ¹³C NMR 16.0, 19.9, 25.5, 32.9, 39.3, 120.2, 128.5, 128.9, 129.5, 133.7, 149.5 ppm.

### Example 9

### Preparation of 3,3'-Di-sec-butyl-5,5',6,6'-tetramethyl-2,2'-biphenol

To a solution of 2-sec-butyl-4,5-dimethylphenol (1.3 g, 7.3 mmol) in 10 mL of dichloromethane was added copper chlorohydroxide-TMEDA complex (10 mg). The solution was stirred for 3 hr at ambient temperature while air was bubbled through. The catalyst (10 mg each) was added at 1 and 2 hours after reaction started. The mixture was diluted with dichloromethane (50 mL) and washed with HCl (0.5N) and water. The solution was then concentrated to give a residue which was further purified by chromatography to afford 3,3'-sec-butyl-5,5',6,6'-tetramethyl-2,2'-biphenol (0.45 g, 35%). ¹H NMR 0.87(m, 6H), 1.21 (d, J = 7 Hz, 6H), 1.65 (m, 4H), 1.85 (m, 6H), 2.26 (s, 6H), 3.01 (m, 2H), 4.57 (s, 2H), 7.02 (s, 2H) ppm. ¹³C NMR 14.2 & 14.3, 17.9 & 18.0, 21.9, 22.1, 31.4 & 32.0, 35.5, 36.0, 121.9, 130.6, 130.9, 132.8, 135.6, 151.2 ppm.

### Example 10

### Preparation of 3,3',6,6'-tetramethyl-5,5'-disec-butyl-2,2'-biphenol

To a solution of 4-sec-butyl-2,5-dimethylphenol (3.9 g, 22 mmol) in 4 mL of dichloromethane was added copper chlorohydroxide-TMEDA complex (40 mg). The solution was stirred for 3 hr at ambient temperature while air was bubbled through. The catalyst (40 mg each) was added at 1 and 2 hours after reaction started. The mixture was diluted with dichloromethane (40 mL) and washed with HCl (0.5 N) and water. The solution was then concentrated to give a residue which was further purified by chromatography and reprecipitated from cool hexanes to afford 3,3',6,6'-tetramethyl-5,5'-di-sec-butyl-2,2'-biphenol (2.1 g, 54 %). ¹H NMR 0.87 (m, 6H), 1.25 (d, J = 7 Hz, 6H), 1.65 (m, 4H), 1.91 (m, 6H), 2.30 (s, 6H), 2.91 (m, 2H), 4.68 (2s, 2H), 7.10 (s, 2H) ppm. ¹³C NMR 11.9 & 12.0, 14.90 & 14.97 & 15.03 & 15.09, 15.7, 21.0 & 21.1, 30.49 & 30.52 & 30.74 & 30.77, 35.6 & 35.7, 119.8, 121.3, 128.0, 132.51 & 132.55 & 132.59 & 132.64, 137.8, 149.0 ppm.

### Example 11

### Preparation of 3,3',5,5'-tetraisopropyl-6,6'-dimethyl-2,2'-biphenol

To a solution of 2,4-diisopropyl-5-methylphenol (50.0 g, 0.26 mol) in 50 mL of dichloromethane was added copper chlorohydroxide-TMEDA complex (5.0 g). The solution was stirred for 18 hr at ambient temperature while air was bubbled through. The mixture was washed with HCl (1.0 N) and extracted with hexanes. The extracts were concentrated to give a residue which was further purified by chromatography to afford 20 g (40%) of 3,3',5,5'-tetraisopropyl-6,6'-dimethyl-2,2'-biphenol. ¹H NMR 1.31 (m, 24H), 1.98 (s, 6H), 3.15 (m, 2H), 3.33 (m, 2H), 4.64 (s, 2H), 7.15 (s, 2H) ppm. ¹³C NMR 17.1, 24.5 & 24.6, 25.5 & 25.7, 29.6, 31.4, 122.5, 125.1, 134.1, 134.2, 141.1, 150.6 ppm.

### Example 12

### Preparation of 3,3'-di-isopropyl-5,5'-dicyclohexyl-6,6'-dimethyl-2,2'-biphenol

To a solution of 4-cyclohexyl-2-isopropyl-5-methylphenol (1.8 g, 7.8 mmol) in 10 mL of dichloromethane was added copper chlorohydroxide-TMEDA complex (20 mg). The solution was stirred for 3 hr at ambient temperature while air was bubbled through. The catalyst (20 mg each) was added at 1 and 2 hours after reaction started. The mixture was diluted with dichloromethane (50 ml) and washed with HCl (0.5N) and water. The solution was then concentrated to give a residue which was further purified by chromatography to afford 3,3'-di-isopropyl-5,5'-dicyclohexyl-6,6'-dimethyl-2,2'-biphenol (1.04 g, 58%). ¹H NMR 1.24 (d, J = 7 Hz, 12H), 1.27 (m, 2H), 1.39 (m, 8H), 1.78 (m, 10H), 1.85 (s, 6H), 2.68 (m, 2H), 2.29 (s, 6H), 3.25 (hept, J = 7 Hz, 2H), 4.60 (s, 2H), 7.12 (s, 2H) ppm. ¹³C NMR 15.1, 22.5, 22.7, 26.4, 27.3, 27.5, 34.0 & 34.3, 40.3, 120.6, 123.8, 131.9, 132.3, 138.3, 148.6 ppm.

### Example 13

### Preparation of 3,3',6,6'-tetramethyl-5,5'-di-cyclohexyl-2,2'-biphenol

A mixture of 2,5-dimethyl-4-cyclohexylphenol (21 g, 0.10 mol), copper chlorohydroxide-TMEDA complex (2.1 g), and methylene chloride (80 mL) was stirred at room temperature for 6 hours while air was bubbled through the mixture. The mixture was washed with HCl (0.5 N) and extracted with hexanes. The extracts were concentrated and dried to give a residue (20 g, which contained 90% of the product, 4-cyclohexyl-2,5-dimethylphenol). The residue was recrystallized from cool hexanes to afford 3,3',6,6'-tetramethyl-5,5'-di-cyclohexyl-2,2'-biphenol (6.5 g, 31 % yield). ¹H NMR (CDCl₃): 1.32 (m, 4H), 1.42 (m, 8H), 1.75-1.90 (m, 8H), 1.93 (s, 6H), 2.28 (s, 6H), 2.70 (m, 2H), 4.60 (s, 2H), 7.13 (s, 2H) ppm. ¹³C NMR (CDCl₃): 15.2, 16.1, 26.4, 27.3, 34.2, 34.1, 40.0, 120.4, 121.5, 128.5, 132.6, 138.3, 149.5 ppm.

### Example 14

### Preparation of 3,3'-di-isopropyl-4,4',5,5',6,6'-hexamethyl-2,2'-biphenol

3,4,5-Trimethylphenol (5g, 37mmol) was dissolved in 30 mL carbon tetrachloride under nitrogen. To this mixture was added scandium triflate (0.9 g) and isopropyl methanesulfonate (6.1g). The mixture was heated to reflux for 3.5 hr under nitrogen. The mixture was poured into water, and the layers were separated. The organic layer was washed with sat. sodium bicarbonate, dried over magnesium sulfate, concentrated, and purified by flash column chromatography on silica gel (eluting with 3% ethyl acetate/hexanes) to give 3 g 2-isopropyl-3,4,5-trimethylphenol(46%). ¹H NMR (CDCl₃) : 6.33 (1H, s), 4.47 (1H,s), 3.36 (1H, quintet, J=12Hz), 2.25 (3H, s), 2.18 (1H, s), 2.10 (1H, s), 1.35 (6H, d, J=12Hz).

2-Isopropyl-3,4,5-trimethylphenol (6g, 34mmol) was dissolved in 10 mL methylene chloride, and 0.4g Cu(OH)Cl-TMEDA was added. The mixture was stirred under ambient air for three hours. Another 0.4 g Cu(OH)Cl-TMEDA was then added, and the mixture stirred for another three hours. To the dark reaction mixture was added 10% HCl solution. The layers were separated, and the organic layer was concentrated, dried over magnesium sulfate, and the residue was chromatographed on silica gel eluting with 5% ether/hexanes to afford 3.1 g (52%) of product as a white solid. ¹H NMR (CDCl₃) : 4.74 (1H, s), 3.37 (1H, quintet, J=7Hz), 2.20 (s, 3H), 2.08 (s, 3H), 1.76 (s, 3H), 1.26(d, 6H, J=7Hz).

### Example 15

### Preparation of 3,3'-diisopropyl-5,5',6,6'-tetramethyl-2,2'-biphenol

A 2-liter resin kettle equipped with mechanical stirrer, dip tube for delivering air, condenser, and receiver was placed in an oil bath and charged with 610 g 4-methylthymol (99% pure by gas chromatography). CuCl (3.05 g) and N,N,N',N'-tetramethylethylenediamine (7.14 g) were charged, and the mixture was heated to 100°C. Air was delivered via dip tube at 1,000 cc/min. After 3.5 hours, the coupling reaction was substantially complete, and the mixture was collected. Gas chromatography showed the mixture to consist of 90% 3,3'-diisopropyl-5,5',6,6'-tetramethyl-2,2'-biphenol, 4% unreacted monomer, and 6% byproducts.

### Example 16

### Preparation of 5,5'-di-t-butyl-3,3'-di-isopropyl-6,6'-dimethyl-2,2'-biphenol

A 500-mL resin kettle equipped with mechanical stirrer, dip tube for delivering air, condenser, and receiver was placed in an oil bath and charged with 4-t-butylthymol (99% pure by gas chromatography). CuCl (1.00 g) and N,N,N',N'-tetramethylethylenediamine (2.35 g) were charged, and the mixture was heated to 100°C. Air was delivered via dip tube at 200 cc/min. After 4 hours, the mixture was collected. Gas chromatography analysis showed the mixture to consist of 78% 5,5'-di-t-butyl-3,3'-diisopropyl-6,6'-dimethyl-2,2'-biphenol, 11% unreacted monomer, and 12% byproducts.

### Example 17

### Preparation of 3,3',5,5'-tetraisopropyl-6,6'dimethyl-2,2'-biphenol

A 22-Liter resin kettle equipped with mechanical stirrer, dip tube for delivering air, condenser, receiver, and electric heating mantle was charged with 7.2 kg 4-isopropylthymol (90% pure by gas chromatography). CuCl (36.5 g) and N,N,N',N'-tetramethylethylenediamine (85.5 g) were charged, and the mixture was heated to 100°C. Air was delivered via dip tube at 5 L/min. After 11 hours, the aeration was stopped, and mixture was allowed to cool for collection. Gas chromatography analysis showed the mixture to consist of 74% 3,3',5,5'-tetraisopropyl-6,6'dimethyl-2,2'-biphenol, 8% unreacted monomer, and 18% byproducts.

## Claims

1. A process for making a compound of the formula 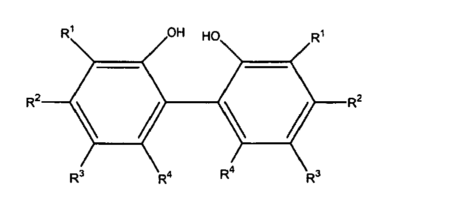 comprising:
oxidatively coupling a compound of the formula 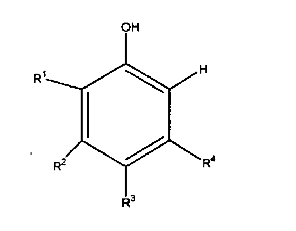 in the presence of a molecular oxygen-containing gas and a copper-containing catalyst, said copper-containing catalyst produced by a process comprising contacting a copper halide salt with an organic diamine compound,
wherein
R1 is C1 to C6 primary, secondary or cyclo alkyl;
R2 is H, C1 to C6 primary, secondary, tertiary or cyclo alkyl;
R3 is C1 to C6 primary, secondary, tertiary or cyclo alkyl; and
R4 is H, C1 to C6 primary, secondary or cyclo alkyl, provided that R2 and R4 are not both H.

2. The process of claim 1 wherein the copper halide salt is CuCl, CuBr, CuI, or CuCl2.

3. The process of claim 2 wherein the organic diamine compound is N,N,N',N'-tetraethylethylene diamine, N,N,N',N'-tetraethyl-1,3-propanediamine, N,N,N',N'-tetraethylmethane diamine , N,N,N',N'-tetramethyl-1,6-hexanediamine, N,N,N',N'-tetramethyl-1,3-propanediamine, dipiperidinomethane, N,N,N',N'-tetramethylethylene diamine or 1,4-diazabicyclo-(2,2,2)-octane.

4. The process of claim 3 wherein R2, R3, and R4 are each methyl or ethyl.

5. A compound of the formula 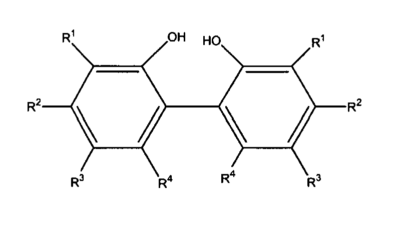 wherein:
R¹ is methyl, ethyl, n-propyl, or isopropyl;
R² is H or methyl;
R³ is methyl, ethyl, n-propyl, isopropyl, or t-butyl; and
R⁴ is methyl;
provided that if R¹ is isopropyl and R² is hydrogen, R³ is other than methyl.

6. A compound of claim 5 wherein
R¹ is methyl or isopropyl;
R² is H or methyl;
R³ is methyl, isopropyl, or t-butyl; and
R⁴ is methyl.

7. A compound of claim 6 wherein
R¹ is isopropyl;
R² is H;
R³ is isopropyl; and
R⁴ is methyl.
